# EUROPEAN PATENT APPLICATION

(11) **EP 4 589 011 A1**
(43) Date of publication of application: **23.07.2025**
(21) Application number: 23865530.2
(22) Date of filing: 12.09.2023
(51) Int. Cl.: C12Q 1/48, C12Q 1/02, G01N 33/50, C07K 14/435, C07K 14/47, C07K 19/00, C12N 15/12, C12N 15/62

(54) **REAGENT FOR MEASURING TYROSINE KINASE ACTIVITY, AND USE THEREOF**

(30) Priority: 12.09.2022 JP 2022144870
(71) Applicant: Hilo Co., Ltd., Sapporo-shi Hokkaido 001-0021 (JP)
(72) Inventor: OHBA Yusuke, Sapporo-shi Hokkaido 060-0808 (JP); AKITA Hirotoshi, Sapporo-shi Hokkaido 060-0808 (JP)
(74) Representative: Wasner, Marita
(86) International application number: PCT/JP2023/033214
(87) International publication number: WO 2024/058178

(57) **Abstract**

The present invention provides a reagent that is for measuring an epidermal growth factor receptor tyrosine kinase activity and that contains: a modified polypeptide to which an acceptor and a donor inducing Förster resonance energy transfer are bound, and which includes CrkL, a CrkL fragment retaining an SH2 domain and a portion subjected to phosphorylation by a tyrosine kinase, or a CrkL variant that has an amino acid sequence having an identity of 90% or more with respect to an amino acid sequence of CrkL or the CrkL fragment and that becomes a substrate for a tyrosine kinase; or a nucleic acid encoding the modified polypeptide. The present invention also provides a method for assessing susceptibility of non-small cell lung cancer cells to a tyrosine kinase inhibitor, a method for preparing non-small cell lung cancer cells that are resistant to a tyrosine kinase inhibitor, and a method for assessing an epidermal growth factor receptor tyrosine kinase activity of non-small cell lung cancer cells, wherein said methods all use the reagent.

## Description

### Field

The present invention relates to a reagent for measuring a tyrosine kinase activity of epidermal growth factor receptor in a lung cancer cell, a method for evaluating sensitivity of a lung cancer cell to a tyrosine kinase inhibitor, a method for preparing a tyrosine kinase inhibitor-resistant lung cancer cell, and a method for measuring a tyrosine kinase activity of epidermal growth factor receptor in a lung cancer cell.

### Background

Lung cancer remains a refractory disease and complete cure is difficult to achieve in numerous cases. Lung cancer is histologically classified into non-small cell lung cancer (NSCLC) and small cell lung cancer (SCLC), and non-small cell lung cancer accounts for approximately 80% of lung cancer cases in Japan.

The epidermal growth factor receptor (EGFR) family is a receptor-type tyrosine kinase protein that plays a central role in regulating signals involved in proliferation, survival, migration, and metastasis of many cancer cells. EGFR gene mutations are driver gene mutations frequently observed in lung cancer, and it is reported that 30-40% of non-small cell lung cancer patients have some form of EGFR gene mutations. Since the early 2000s, tyrosine kinase inhibitors (TKIs) have been used as molecular targeted drugs against EGFR in the treatment of non-small cell lung cancer.

Treatment using molecular targeted drugs, while expected to have high safety and efficacy due to their specificity, is accompanied by problems in that expected therapeutic efficacy is difficult to achieve in patients having mutations in target molecules, thereby making prediction of efficacy and side effects difficult. In the treatment of non-small cell lung cancer, information regarding sensitivity/resistance to TKIs is of significant importance in selection of treatment methods.

For predicting the effect of TKIs before initiation of treatment, determination of treatment strategies has been attempted based on mutations detected by EGFR gene mutation testing using non-small cell lung cancer specimens from patients. However, since TKI-resistant cells contained in tumor tissue are present in extremely small numbers at the initial stage of treatment, detection of TKI resistance mutations is frequently difficult. Furthermore, with respect to unreported mutations, such mutations, even when detected, cannot be immediately utilized for efficacy determination.

The present inventors have created, as a reagent for measuring tyrosine kinase activity of BCR-ABL expressed by Philadelphia chromosome translocation that occurs in chronic myeloid leukemia and some acute lymphoblastic leukemia, a biosensor that induces intramolecular Förster resonance energy transfer utilizing CrkL (Crk Like proto-oncogene, adaptor protein), which is phosphorylated by BCR-ABL (see, for example, Patent Documents 1 and 2). This biosensor enables evaluation of leukemia cell sensitivity to TKIs.

### Citation List

### Patent Literature

Patent Literature 1: Japanese Patent No. 5665262.
Patent Literature 2: Japanese Patent No. 6473080.

### Summary

### Problem to be solved

The present inventors have found that a biosensor that induces intramolecular Förster resonance energy transfer can be utilized in evaluation of EGFR tyrosine kinase activity and sensitivity to TKIs in lung cancer cells.

The present disclosure provides the followings.

[Item 1] A reagent for measuring a tyrosine kinase activity of EGFR, containing: a modified polypeptide, which contains CrkL; a CrkL fragment having an SH2 domain and a portion subjected to phosphorylation by a tyrosine kinase; or a CrkL variant consisting of an amino acid sequence having an identity of 90% or more with the amino acid sequence of CrkL or the CrkL fragment and acting as a substrate for a tyrosine kinase, wherein said modified polypeptide further has a donor and an acceptor that induce Förster resonance energy transfer bound thereto; or a nucleic acid encoding the modified polypeptide.

[Item 1'] A reagent for measuring a tyrosine kinase activity ofEGFR, containing: a modified polypeptide containing a donor and an acceptor that induce Förster resonance energy transfer bound to CrkL, a CrkL fragment having an SH2 domain and a portion subjected to phosphorylation by a tyrosine kinase, or a CrkL variant consisting of an amino acid sequence having an identity of 90% or more with the amino acid sequence of CrkL or the CrkL fragment and acting as a substrate for a tyrosine kinase; or a nucleic acid encoding the modified polypeptide.

[Item 2] The reagent according to Item 1 or 1', wherein the CrkL is a polypeptide consisting of the amino acid sequence represented by SEQ **ID** NO: 1.

[Item 3] The reagent according to Item 1 or 1', wherein the CrkL fragment is a polypeptide consisting of an amino acid sequence in which 1 to 81 arbitrary amino acids are deleted from the C-terminus of the amino acid sequence represented by SEQ **ID** NO: 1.

[Item 4] The reagent according to Item 1 or 1', wherein the CrkL variant is any one of the following polypeptides (1) to (3): (1) a polypeptide consisting of an amino acid sequence in which at least one amino acid of amino acids at positions 91 to 97 in the amino acid sequence represented by SEQ ID NO: 1 is substituted; (2) a polypeptide consisting of an amino acid sequence in which 1 to 81 arbitrary amino acids are deleted from the C-terminus of the amino acid sequence represented by SEQ **ID** NO: 1, and at least one amino acid of amino acids at positions 91 to 97 is substituted; or (3) a polypeptide consisting of an amino acid sequence having an identity of 90% or more with the amino acid sequence of the polypeptide of (1) or (2), and having suppressed cleavage compared to a polypeptide consisting of an amino acid sequence in which amino acids at positions 91 to 97 are not substituted, wherein the polypeptide acts as a substrate for a tyrosine kinase.

[Item 5] The reagent according to Item 4, wherein the substitution of at least one amino acid of amino acids at positions 91 to 97 is a substitution of the amino acid at position 94.

[Item 6] The reagent according to Item 4, wherein the substitution of at least one amino acid of amino acids at positions 91 to 97 is a substitution of the amino acid at position 94 with alanine.

[Item 7] The reagent according to any one of Items 1, 1' and 2 to 6, wherein the donor and the acceptor are fluorescent proteins.

[Item 8] The reagent according to any one of Items 1, 1' and 2 to 7, wherein the modified polypeptide has a structure in which a fluorescent protein as the donor is bound, directly or via a linker sequence, to one terminus of the CrkL, the CrkL fragment, or the CrkL variant, and a fluorescent protein as the acceptor is bound, directly or via a linker sequence, to the other terminus.

[Item 9] The reagent according to any one of Items 1, 1' and 2 to 8, wherein the donor is CFP or a variant thereof, and the acceptor is YFP or a variant thereof.

[Item 10] The reagent according to any one of Items 1, 1' and 2 to 9, wherein the donor is ECFP, and the acceptor is m1Venus.

[Item 11] The reagent according to any one of Items 1, 1' and 2 to 10, wherein the modified polypeptide further contains a nuclear export signal.

[Item 12] The reagent according to Item 1 or 1', wherein the modified polypeptide has an amino acid sequence represented by any one of SEQ ID NOs: 4 to 7.

[Item 13] A method for evaluating sensitivity of a lung cancer cell to a TKI, including the steps of: (a) contacting the modified polypeptide as defined in any one of Items 1, 1' and 2 to 12 with EGFR of the lung cancer cell in the presence of the TKI; (b) detecting fluorescence from the modified polypeptide; and (c) evaluating sensitivity to the TKI based on the fluorescence.

[Item 14] The method according to Item 13, wherein step (a) is performed within the lung cancer cell.

[Item 15] The method according to Item 13 or 14, wherein step (a) is performed by incubating a lung cancer cell introduced with a nucleic acid encoding the modified polypeptide in the presence of the TKI.

[Item 16] The method according to Item 15, wherein introduction of the nucleic acid is performed by lipofection.

[Item 17] The method according to any one of Items 13 to 16, wherein in step (b), fluorescence at the emission wavelength of the acceptor is detected, and in step (c), when intensity of the fluorescence is lower than intensity of fluorescence from the modified polypeptide at said wavelength in the absence of the TKI, or when the intensity is lower than a predetermined cutoff value, the lung cancer cell is evaluated as being sensitive to the TKI.

[Item 18] The method according to any one of Items 13 to 16, wherein in step (b), fluorescence at the emission wavelength of the acceptor is detected, and in step (c), when intensity of the fluorescence is equal to or higher than intensity of fluorescence from the modified polypeptide at said wavelength in the absence of the TKI, or when the intensity is equal to or higher than a predetermined cutoff value, the lung cancer cell is evaluated as being resistant to the TKI.

[Item 19] The method according to any one of Items 13 to 16, wherein in step (b), fluorescence at the emission wavelength of the donor is detected, and in step (c), when intensity of the fluorescence is higher than intensity of fluorescence from the modified polypeptide at said wavelength in the absence of the TKI, or when the intensity is higher than a predetermined cutoff value, the lung cancer cell is evaluated as being sensitive to the TKI.

[Item 20] The method according to any one of Items 13 to 16, wherein in step (b), fluorescence at the emission wavelength of the donor is detected, and in step (c), when intensity of the fluorescence is equal to or lower than intensity of fluorescence from the modified polypeptide at said wavelength in the absence of the TKI, or when the intensity is equal to or lower than a predetermined cutoff value, the lung cancer cell is evaluated as being resistant to the TKI.

[Item 21] The method according to any one of Items 13 to 16, wherein in step (b), fluorescence from the modified polypeptide at emission wavelengths of the donor and the acceptor is detected, and in step (c), when a ratio of fluorescence intensity at the emission wavelength of the acceptor to fluorescence intensity at the emission wavelength of the donor is lower than a ratio of fluorescence intensity at the emission wavelength of the acceptor to fluorescence intensity at the emission wavelength of the donor in the absence of the TKI, or when the ratio is lower than a predetermined cutoff value, the lung cancer cell is evaluated as being sensitive to the TKI.

[Item 22] The method according to any one of Items 13 to 16, wherein in step (b), fluorescence from the modified polypeptide at emission wavelengths of the donor and the acceptor is detected, and in step (c), when a ratio of fluorescence intensity at the emission wavelength of the acceptor to fluorescence intensity at the emission wavelength of the donor is equal to or higher than a ratio of fluorescence intensity at the emission wavelength of the acceptor to fluorescence intensity at the emission wavelength of the donor in the absence of the TKI, or when the ratio is equal to or higher than a predetermined cutoff value, the lung cancer cell is evaluated as being resistant to the TKI.

[Item 23] A method for preparing a TKI-resistant lung cancer cell, including the steps of: (a) contacting the modified polypeptide as defined in any one of Items 1, 1' and 2 to 12 with EGFR of the lung cancer cell in the presence of the TKI; (b) detecting fluorescence from the modified polypeptide; (c) evaluating sensitivity to the TKI based on the fluorescence; and (d) collecting a cell evaluated as being resistant to the TKI.

[Item 24] The method according to Item 23, wherein step (a) is performed within the lung cancer cell.

[Item 25] The method according to Item 23 or 24, wherein step (a) is performed by incubating a lung cancer cell introduced with a nucleic acid encoding the modified polypeptide in the presence of the TKI.

[Item 26] The method according to Item 25, wherein introduction of the nucleic acid is performed by lipofection.

[Item 27] The method according to any one of Items 23 to 26, wherein in step (b), fluorescence at the emission wavelength of the acceptor is detected, and in step (c), when intensity of the fluorescence is equal to or higher than intensity of fluorescence from the modified polypeptide at said wavelength in the absence of the TKI, or when the intensity is equal to or higher than a predetermined cutoff value, the lung cancer cell is evaluated as being resistant to the TKI.

[Item 28] The method according to any one of Items 23 to 26, wherein in step (b), fluorescence at the emission wavelength of the donor is detected, and in step (c), when intensity of the fluorescence is equal to or lower than intensity of fluorescence from the modified polypeptide at said wavelength in the absence of the TKI, or when the intensity is equal to or lower than a predetermined cutoff value, the lung cancer cell is evaluated as being resistant to the TKI.

[Item 29] The method according to any one of Items 23 to 26, wherein in step (b), fluorescence from the modified polypeptide at emission wavelengths of the donor and the acceptor is detected, and in step (c), when a ratio of fluorescence intensity at the emission wavelength of the acceptor to fluorescence intensity at the emission wavelength of the donor is equal to or higher than a ratio of fluorescence intensity at the emission wavelength of the acceptor to fluorescence intensity at the emission wavelength of the donor in the absence of the TKI, or when the ratio is equal to or higher than a predetermined cutoff value, the lung cancer cell is evaluated as being resistant to the TKI.

[Item 30] A method for measuring a tyrosine kinase activity in a lung cancer cell, including the steps of: (a') contacting the modified polypeptide as defined in any one of Items 1, 1' and 2 to 12 with EGFR of the lung cancer cell; and (b) detecting fluorescence from the modified polypeptide.

[Item 31] The method according to Item 30, wherein step (a') is performed within the lung cancer cell.

[Item 32] The method according to Item 30 or 31, wherein step (a') is performed by incubating a lung cancer cell introduced with a nucleic acid encoding the modified polypeptide in the presence of the TKI.

[Item 33] The method according to Item 32, wherein introduction of the nucleic acid is performed by lipofection.

[Item 34] The method according to any one of Items 30 to 33, wherein in step (b), fluorescence from the modified polypeptide at the emission wavelength of the donor and/or the acceptor is detected.

### Advantageous Effects of Invention

According to the present invention, it is possible to evaluate EGFR tyrosine kinase activity of lung cancer cells, particularly non-small cell lung cancer cells, and their sensitivity to TKIs. Further, according to the present invention, it is possible to evaluate sensitivity to TKIs of individual cells in a cell population containing lung cancer cells derived from lung cancer patients, particularly in a cell population containing non-small cell lung cancer cells derived from lung cancer patients, thereby enabling detection and isolation of a small number of TKI-resistant cells present in the cell population.

### Brief Description of Drawings

[FIG.1] Fig. 1 is a set of graphs showing changes in FRET efficiency upon EGF stimulation in Cos-1 cells expressing Pickles, an example of the modified polypeptides of the present disclosure. (A) shows comparison with Picchu, a Crkll biosensor, (B) shows the effect of AG1478 treatment, and (C) shows comparison with HGF stimulation. In all graphs, FRET efficiency normalized to the mean value before EGF stimulation is plotted.
[FIG.2] Fig. 2 is a graph showing changes in FRET efficiency upon EGF stimulation in HCC827 cells expressing Pickles_2.34 and Pickles_2.34NES, examples of the modified polypeptides of the present disclosure.
[FIG.3] Fig. 3 is a set of graphs showing changes in FRET efficiency in lung cancer cells expressing Pickles_2.34NES. (A) shows comparison between A549 cells and H1299 cells, (B) shows the effect of Gefitinib treatment in HCC827 cells, PC3 cells, PC9 cells, and H1975 cells, and (C) shows the effect of Afatinib treatment in HCC827 cells. In graphs (B) and (C), FRET efficiency normalized to the mean value before EGF stimulation is plotted.
[FIG.4] Figure 4 is a set of views showing the efficiency of transfecting lung cancer cells with the Pickles_2.34NES expression vector using FuGene HD (FGHD in figure), polyethylenimine (PEI in figure), Lipofectamine^{®} 2000 (LF2000 in figure), Lipofectamine^{®} 3000 (LF3000 in figure), and electroporation (Amaxa in figure). (A) shows representative fluorescence images of HCC827 cells, (B) shows a graph of transfection efficiency and CFP fluorescence intensity in HCC827 cells, and (C) shows FSC-SSC plots and CFP-FRET plots of PC3 cells after transfection.
[FIG. 5]. Figure 5 is a set of plots showing CFP-FRET plots (top four panels) and FSC-SSC plots (bottom panel) of lung cancer cells expressing Pickles_2.34 NES with or without Afatinib treatment.
[FIG. 6]. Figure 6 is a graph showing Afatinib sensitivity/resistance of H cells and L cells isolated from Pickles_2.34 NES-expressing lung cancer cell populations based on changes in FRET efficiency upon Afatinib treatment.
[FIG. 7]. Figure 7 is a set of graphs showing the relationship between treatment concentration of Gefitinib, Erlotinib, Afatinib, or Osimertinib and FRET efficiency in lung cancer cells expressing Pickles_2.34 NES.

### Detailed Description of Invention

The following description may be based on representative embodiments or specific examples, but the present invention is not limited to such embodiments or specific examples. The upper limit value and the lower limit value of each numerical range shown in this specification can be combined as desired. In this specification, a numerical range represented using "to" or "-" means a range that includes both end values as an upper limit value and a lower limit value, unless otherwise noted.

In the present disclosure, amino acid sequence identity means the percentage (%) of identical amino acids relative to all overlapping amino acids in the optimal alignment calculated using algorithms known in the art (preferably, the algorithm may consider introducing gaps into one or both sequences for optimal alignment). Identity can be calculated, for example, by aligning two amino acid sequences using NCBI BLAST-2 (National Center for Biotechnology Information Basic Local Alignment Search Tool) with default settings. When a sequence to be aligned contains a modified amino acid or amino acid analog, the identity calculation is performed using the sequence where the modified amino acid or amino acid analog has been replaced with its corresponding natural amino acid.

### CrkL, CrkL Fragment, CrkL Variant

In the present disclosure, the modified polypeptide contains CrkL, a CrkL fragment having an SH2 domain and a portion subjected to phosphorylation by a tyrosine kinase, or a CrkL variant consisting of an amino acid sequence having an identity of 90% or more with the amino acid sequence of CrkL or the CrkL fragment and acting as a substrate for a tyrosine kinase. The modified polypeptide containing CrkL, a CrkL fragment, or a CrkL variant means that the amino acid sequence of the modified polypeptide contains the amino acid sequence of CrkL, the amino acid sequence of a CrkL fragment, or the amino acid sequence of a CrkL variant.

CrkL is an adapter protein of 303 residues in total having one SH2 domain and two SH3 domains, and a tyrosine residue present in an intervening sequence between the two SH3 domains is subjected to phosphorylation. Said tyrosine residue is positioned at residue 207 in the amino acid sequence of CrkL represented by SEQ ID NO: 1 (UniProt accession number P46109).

In the present disclosure, the CrkL fragment is a polypeptide consisting of a partial amino acid sequence of CrkL that has the SH2 domain and the portion subjected to phosphorylation by a tyrosine kinase. An example of the CrkL fragment is a polypeptide consisting of an amino acid sequence in which 1 to 81 arbitrary amino acids are deleted from the C-terminus of the amino acid sequence represented by SEQ ID NO: 1. This polypeptide can also be described as a polypeptide consisting of a partial amino acid sequence of CrkL represented by SEQ ID NO: 1 that has amino acids 1 to 222 in the amino acid sequence represented by SEQ ID NO: 1.

The CrkL variant is a polypeptide consisting of an amino acid sequence having an identity of 70% or more, preferably 80% or more, more preferably 90% or more, still more preferably 95% or more, particularly preferably 97%, 98% or 99% or more with the amino acid sequence of CrkL, or an amino acid sequence having an identity of 70% or more, preferably 80% or more, more preferably 90% or more, still more preferably 95% or more, particularly preferably 97%, 98% or 99% or more with the amino acid sequence of the CrkL fragment, and acting as a substrate for a tyrosine kinase.

One example of the CrkL variant is a polypeptide consisting of an amino acid sequence in which at least one amino acid of amino acids at positions 91 to 97 in the amino acid sequence represented by SEQ **ID** NO: 1 is substituted (referred to as polypeptide 1).

Another example of the CrkL variant is a polypeptide consisting of an amino acid sequence in which 1 to 81 arbitrary amino acids are deleted from the C-terminus of the amino acid sequence represented by SEQ **ID** NO: 1, and at least one amino acid of amino acids at positions 91 to 97 is substituted (referred to as polypeptide 2).

Still another example of the CrkL variant is a polypeptide consisting of an amino acid sequence having an identity of 90% or more with the amino acid sequence of polypeptide 1 or polypeptide 2, and having suppressed cleavage compared to a polypeptide consisting of an amino acid sequence in which amino acids at positions 91 to 97 are not substituted, wherein the polypeptide acts as a substrate for a tyrosine kinase (referred to as polypeptide 3).

In the amino acid sequences of polypeptides 1 to 3, the substitution of at least one amino acid of amino acids at positions 91 to 97 is preferably a substitution of the amino acid at position 94. In polypeptides 1 to 3, by substituting at least one amino acid from positions 91 to 97 from the N-terminus, preferably the amino acid at position 94 from the N-terminus, cleavage by intracellular proteases is suppressed compared to when such substitution is not present. Therefore, a modified polypeptide containing polypeptides 1 to 3 and having a donor and an acceptor that induce Förster resonance energy transfer bound thereto can avoid fluorescence attenuation due to cleavage by intracellular proteases when introduced into or expressed in cells.

The amino acid substitution may be a substitution with chemically similar amino acid residues, so-called conservative substitution. Examples of conservative substitutions include substitutions between amino acids such as glycine (Gly) and proline (Pro), glycine and alanine (Ala) or valine (Val), leucine (Leu) and isoleucine (Ile), glutamic acid (Glu) and glutamine (Gln), aspartic acid (Asp) and asparagine (Asn), cysteine (Cys) and threonine (Thr), threonine and serine (Ser) or alanine, and lysine (Lys) and arginine (Arg).

The number of amino acids to be substituted is, for example, 1 to 30, 1 to 25, 1 to 22, 1 to 20, 1 to 15, 1 to 12, 1 to 10, 1 to 8, 1 to 6, 1 to 5, 1 to 4, 1 to 3, 1 to 2, or 1.

In the amino acid sequences of polypeptides 1 to 3, the substitution of at least one amino acid of amino acids at positions 91 to 97 is preferably a substitution of one amino acid of amino acids at positions 91 to 97, more preferably a substitution of the amino acid at position 94 with alanine.

### Donor and Acceptor that Induce Förster Resonance Energy Transfer

Förster resonance energy transfer is an interaction between two or more molecules in different electronic excited states, in which energy is transferred from one molecule (donor) excited by an external light source or energy generated by a chemical reaction to another molecule (acceptor). Förster resonance energy transfer includes fluorescence resonance energy transfer (FRET) using fluorescent substances as the donor and acceptor, and bioluminescence resonance energy transfer (BRET) using a bioluminescent substance and a fluorescent substance as the donor and acceptor, respectively.

As the donor and acceptor that induce Förster resonance energy transfer, different luminescent substances are used. The luminescent substances can be either luminescent compounds or luminescent proteins. Further, to cause Förster resonance energy transfer, the donor must be a substance capable of transferring energy to the acceptor through resonance of excited electrons.

Examples of combinations of donor and acceptor that induce Förster resonance energy transfer include, in the order of donor and acceptor: a combination of a fluorescent compound and a fluorescent compound, a fluorescent compound and a fluorescent protein, a fluorescent protein and a fluorescent compound, a fluorescent protein and a fluorescent protein, a bioluminescent protein and a fluorescent compound, and a bioluminescent protein and a fluorescent protein.

Examples of fluorescent compounds include: fluorescein such as carboxyfluorescein, 6-(fluorescein)-5,6-carboxamidohexanoic acid, or fluorescein isothiocyanate; Alexa Fluor dye such as Alexa Fluor 488 and Alexa Fluor 594; cyanine dye such as Cy2, Cy3, Cy5, and Cy7; coumarin; R-phycoerythrin; allophycocyanin; modified allophycocyanin such as XL665; Texas Red; Princeton Red; phycobiliprotein; europium cryptate; avidin; streptavidin; rhodamine; eosin; erythrosin; naphthalene; pyrene; pyridyloxazole; benzoxadiazole and sulfoindocyanine; derivatives thereof; and complexes thereof.

When using fluorescent compounds as the donor and acceptor that induce Förster resonance energy transfer, examples of combinations of donor and acceptor include, in the order of donor and acceptor: rhodamine B sulfonyl chloride and fluorescein maleimide; N-iodoacetyl-N'-(5-sulfo-1-naphthyl)ethylenediamine (1,5-IAEDANS) or iodoacetamide and succinimidyl 6-(N-(7-nitrobenzo-2-oxa-1,3-diazol-4-yl)amino)hexanoate (NBD-X,SE); (diethylamino)coumarin (DEAC) or N-methylanthraniloyl deoxyguanine nucleotide (such as Mant dGDP or Mant dGTP) and sNBD (succinimidyl 6-[(7-nitrobenzo-2-oxa-1,3-diazol-4-yl)amino]hexanoate).

One example of fluorescent proteins is GFP (Green Fluorescent Protein) and variants thereof. To date, various GFP variants having different excitation wavelengths or fluorescence wavelengths from GFP have been developed, and examples that can be used include BFP, CFP, YFP, and variants thereof. Another example of fluorescent proteins includes DsRed, Midoriishi-Cyan (MiCy), Kusabira-Orange (KO), and its monomer mKO.

The fluorescent proteins may be variants with modified amino acid sequence order, such as circular permutation variants. For example, a circular permutation variant of ECFP (called cp173-ECFP) in which the amino acid sequence from positions 1 to 172 and the amino acid sequence from positions 173 to 238 are swapped has enhanced fluorescence intensity and can be suitably used as a fluorescent protein in the present disclosure.

Examples of bioluminescent proteins include firefly luciferin, cypridina luciferin, Renilla luciferin, bacterial luciferin, krill luciferin, dinoflagellate luciferin, Latia luciferin, earthworm luciferin, and derivatives of these luciferins.

When using fluorescent proteins as the donor and acceptor that induce Förster resonance energy transfer, examples of combinations of donor and acceptor include, in the order of donor and acceptor: Sirius and mseCFP, EBFP and C-S65T, mTFP1 and eYFP, T-Sapphire and mOrange, mAmetrine and tdTomato, T-Sapphire and PSmOrange2, mCellurian and mCitrine, mTurquoise and eYFP, eCFP and tdTomato, eCFP and mDsRed, eCFP and YPet, CyPet and YPet, LSSmOrange and LSSmKate2, mTurquiose and cpVenus, CFP and YFP, CFP and cpVenus, mseCFP and PA-GFP, mTFP1 and mCitrine, GFP and TMR, GFP and Alexa546, eGFP and mStrawberry, eGFP and Halo-TMR, Alexa488 and Alexa594, Alexa488 and TMR, eGFP and mDsRed, eGFP and mRFP, sYFP and mRFP, TagGFP and TagRFP, TMR and Atto647, Cy3 and Atto647N, TMR and Cy5, TMR and IC5, Clover and mRuby2, Cy3 and Cy5, mKO and mCherry, tagRFP and mPlum, mOrange2 and mKate2, mOrange and mCherry, Qdot and Cy3, QDot525 and Cy3, RLuc and EYFP, RLuc8 and EYFP, RLucX and Venus, cpeCFP and mVenus.

The combination of donor and acceptor that induce Förster resonance energy transfer is preferably a combination of CFP or a variant thereof and YFP or a variant thereof, and more preferably a combination of cp173-ECFP and a Venus variant (called m1Venus) in which Ala at position 207 in the amino acid sequence of Venus, which is a variant of YFP, is substituted with Lys.

### Modified Polypeptide

In the present disclosure, the modified polypeptide has two or more luminescent substances that induce Förster resonance energy transfer, and these luminescent substances are in a donor-acceptor relationship. In one embodiment, the modified polypeptide is a polypeptide having a donor and an acceptor that induce Förster resonance energy transfer bound to CrkL, a CrkL fragment, or a CrkL variant.

The modified polypeptide preferably has a structure in which one or more luminescent substances as the donor are bound to one terminus of CrkL, a CrkL fragment, or a CrkL variant, and one or more luminescent substances as the acceptor are bound to the other terminus, either directly or via a linker sequence. The donor and acceptor may be at either the N-terminus or C-terminus of CrkL, the CrkL fragment, or the CrkL variant, and their positions may be interchanged with each other.

When using fluorescent compounds as the donor and acceptor that induce Förster resonance energy transfer, binding (modification) of fluorescent compounds to CrkL, a CrkL fragment, or a CrkL variant can be performed using protein chemical modification methods known to those skilled in the art (such as cross-linking). Additionally, a tag sequence having binding ability to fluorescent compounds (such as HaloTag^{®}) can be added to CrkL, the CrkL fragment, or the CrkL variant, and fluorescent compounds can be bound to CrkL, the CrkL fragment, or the CrkL variant via the tag sequence.

When using fluorescent proteins as the donor and acceptor that induce Förster resonance energy transfer, the fluorescent proteins can be bound to CrkL, the CrkL fragment, or the CrkL variant either directly or via a linker sequence. The linker sequence may be any sequence that does not interfere with Förster resonance energy transfer, and may be, for example, a peptide consisting of 1 to 10 amino acids, 1 to 5 amino acids, or 1 to 3 amino acids. Examples of linker sequences include GGS, RGR, CGR, GGR, LE, and GGSGG (SEQ ID NO: 2).

The modified polypeptide may also contain a nuclear export signal (NES). The NES consists of an amino acid sequence represented by LxxxLxxLxL (where L means a hydrophobic amino acid such as leucine, isoleucine, or valine, and x means any amino acid). A preferred example of NES is the NES from protein kinase A inhibitor, which has the amino acid sequence LALKLAGLDI (SEQ ID NO: 3).

One preferred example of the modified polypeptide in the present disclosure is a polypeptide (called Pickles_2.3) having, from the N-terminus in this order, the amino acid sequence of Venus, the amino acid sequence corresponding to residues 1-222 of CrkL, and the amino acid sequence of cp173-ECFP. The amino acid sequence of Pickles_2.3 is shown in SEQ ID NO: 4.

Another preferred example of the modified polypeptide in the present disclosure is a polypeptide (called Pickles_2.31) having, from the N-terminus in this order, the amino acid sequence of m1Venus, the amino acid sequence corresponding to residues 1-222 of CrkL, and the amino acid sequence of cp173-ECFP. The amino acid sequence of Pickles_2.31 is shown in SEQ ID NO: 5.

Another preferred example of the modified polypeptide in the present disclosure is a polypeptide (called Pickles_2.34) having, from the N-terminus in this order, the amino acid sequence of m1Venus, the amino acid sequence corresponding to residues 1-222 of CrkL in which aspartic acid at position 94 is substituted with alanine, and the amino acid sequence of cp173-ECFP. The amino acid sequence of Pickles_2.34 is shown in SEQ ID NO: 6.

Another preferred example of the modified polypeptide in the present disclosure is a polypeptide having the NES from protein kinase A inhibitor added to the N-terminus of Pickles_2.34, namely, a polypeptide (called Pickles_2.34NES) having, from the N-terminus in this order, the amino acid sequence of NES from protein kinase A inhibitor, the amino acid sequence of m1Venus, the amino acid sequence corresponding to residues 1-222 of CrkL in which aspartic acid at position 94 is substituted with alanine, and the amino acid sequence of cp173-ECFP. The amino acid sequence of Pickles_2.34NES is shown in SEQ ID NO: 7.

When the tyrosine residue contained in the portion of the modified polypeptide subjected to phosphorylation by a tyrosine kinase is not phosphorylated, fluorescence from the donor is detected by irradiating the modified polypeptide with light of a wavelength that excites the donor or by adding a substrate that causes the donor to emit light. On the other hand, when the tyrosine residue contained in the portion of the modified polypeptide subjected to phosphorylation by a tyrosine kinase is phosphorylated, fluorescence from the acceptor is detected by irradiating the modified polypeptide with light of a wavelength that excites the donor or by adding a substrate that causes the donor to emit light. This phenomenon is understood to occur because the SH2 domain recognizes and binds to the phosphorylated tyrosine residue, causing a conformational change in the modified polypeptide, which brings the donor and acceptor into close proximity, resulting in Förster resonance energy transfer.

In this way, tyrosine kinase activity of EGFR can be measured by evaluating the degree of phosphorylation of the modified polypeptide contacted with EGFR based on the decrease in donor fluorescence, increase in acceptor fluorescence, or increase in the ratio of acceptor fluorescence intensity to donor fluorescence intensity. In one aspect, the present invention provides a method for measuring tyrosine kinase activity of EGFR in lung cancer cells, including the steps of: a') contacting the above-described modified polypeptide with EGFR of lung cancer cells, preferably EGFR of non-small cell lung cancer cells, and b) detecting fluorescence from the modified polypeptide. Step a') is as described below except that TKI is not present, and step b) is as described below. In one embodiment, steps a') and b) are performed in vitro or ex vivo.

### Nucleic Acid Encoding the Modified Polypeptide

The modified polypeptide can be produced by genetic engineering methods using a nucleic acid encoding the modified polypeptide.

The nucleic acid may be either DNA or mRNA, and when it is DNA, it is preferably in the form of an expression vector. The expression vector contains DNA encoding the modified polypeptide operably linked to regulatory sequences such as a promoter. The expression vector contains a replication origin and a promoter, and may also contain, as needed, an enhancer, a transcription termination sequence (terminator), a ribosome binding site, a polyadenylation signal, and the like.

Examples of expression vectors that can be used in the present disclosure include a plasmid vector, a viral vector (such as a retroviral vector, a lentiviral vector, a Sendai virus vector, an adenoviral vector, and an adeno-associated viral vector), and an **RNA** vector.

**In** one embodiment, the modified polypeptide can be produced by introducing an expression vector containing **DNA** encoding the modified polypeptide into a suitable host cell, such as Escherichia coli, an insect cell, and an animal cell, and expressing it therein. Furthermore, by using, as a host cell, a lung cancer cell itself whose **EGFR** tyrosine kinase activity is desired to be measured or whose sensitivity to **TKI** is desired to be evaluated, the modified polypeptide can be expressed within the lung cancer cell while being contacted with EGFR.

Genetic engineering methods, including preparation of DNA and expression vectors, selection of host cells and methods for introducing expression vectors thereinto, expression and purification of proteins, and the like, are well known to those skilled in the art and can be carried out according to instructions provided in experimental operation manuals describing various techniques in detail.

In another embodiment, the modified polypeptide can also be produced by performing cell-free protein synthesis using **DNA or mRNA** encoding the modified polypeptide. Cell-free protein synthesis systems include systems utilizing cell extracts from cells such as Escherichia coli, wheat germ, yeast, rabbit reticulocytes, insect cells, and mammalian cultured cells, and reconstituted systems composed of factors necessary for protein synthesis.

When using fluorescent compounds as the donor and acceptor that induce Förster resonance energy transfer, the modified polypeptide can be produced by producing CrkL, a CrkL fragment, a CrkL variant, or a polypeptide having a tag sequence added thereto by genetic engineering methods, and binding fluorescent compounds to the obtained polypeptide by chemical modification methods.

In one aspect, the present disclosure provides a reagent for measuring tyrosine kinase activity of EGFR, containing: a modified polypeptide, which contains CrkL; a CrkL fragment having an SH2 domain and a portion subjected to phosphorylation by a tyrosine kinase; or a CrkL variant consisting of an amino acid sequence having an identity of 90% or more with the amino acid sequence of CrkL or the CrkL fragment and acting as a substrate for a tyrosine kinase, wherein said modified polypeptide further has a donor and an acceptor that induce Förster resonance energy transfer bound thereto; or a nucleic acid encoding the modified polypeptide. The modified polypeptide and the nucleic acid encoding the modified polypeptide are as described above.

In one aspect, the present disclosure provides a kit for measuring tyrosine kinase activity of EGFR. The kit contains the modified polypeptide or the nucleic acid encoding the modified polypeptide, and can further contain other reagents such as reagents for introducing the modified polypeptide or the nucleic acid encoding the modified polypeptide into a lung cancer cell (for example, transfection reagents) and buffers. The kit may further contain other components such as instruments used for measuring EGFR tyrosine kinase activity (for example, vessels for containing lung cancer cells) and instruction manuals regarding methods of use.

### Method for Evaluating Sensitivity to TKI

In one aspect, the present disclosure provides a method for evaluating sensitivity of a lung cancer cell, particularly a non-small cell lung cancer cell, to a TKI, including the steps of: a) contacting the above-described modified polypeptide with EGFR of the lung cancer cell in the presence of the TKI; b) detecting fluorescence from the modified polypeptide; and c) evaluating sensitivity to the TKI based on the fluorescence. In one embodiment, steps a) to c) are performed in vitro or ex vivo.

The lung cancer cell may be either a small cell lung cancer cell or a non-small cell lung cancer cell, and is preferably a non-small cell lung cancer cell. Further, the lung cancer cell may be either a cultured cell line or a cell obtained from a lung cancer patient.

The TKI may be any TKI having the ability to inhibit a tyrosine kinase activity of EGFR, and examples thereof include imatinib, dasatinib, gefitinib, erlotinib, osimertinib, nilotinib, bosutinib, afatinib, and AG1478.

In one embodiment, step a) is performed within a lung cancer cell whose sensitivity to the TKI is desired to be evaluated. Step a) can be performed by incubating, in the presence of the TKI, a lung cancer cell introduced with the modified polypeptide or a lung cancer cell introduced with a nucleic acid encoding the modified polypeptide, preferably a lung cancer cell introduced with a nucleic acid encoding the modified polypeptide.

Introduction of the modified polypeptide into the lung cancer cell can be performed using commercially available protein introduction reagents such as BioPORTER^{®} Protein Delivery Reagent (GEN) and SN21-LK15 Intracellular Delivery Peptide (CPA) according to the manufacturer's protocol.

Introduction of a nucleic acid encoding the modified polypeptide into the lung cancer cell can be performed by known methods such as: chemical introduction methods including lipofection using cationic lipids such as Lipofectamine^{®} 2000 and Lipofectamine^{®} 3000, DEAE-dextran method, calcium phosphate co-precipitation method, polyethylenimine method; biological introduction methods using viral vectors; and physical introduction methods including electroporation, particle gun, microinjection. The introduction of the nucleic acid is preferably performed by lipofection.

Incubation of the lung cancer cell in the presence of the TKI can be performed by maintaining a lung cancer cell introduced with the modified polypeptide or a lung cancer cell introduced with a nucleic acid encoding the modified polypeptide together with the TKI under conditions where the cell can be kept alive, for example, by maintaining said lung cancer cell in a suitable medium or buffer containing the TKI.

In another embodiment, step a) can be performed by incubating a lysate of the lung cancer cell or a fraction containing EGFR prepared from the lung cancer cell in the presence of the TKI.

In step b), to cause the donor to emit light, light of a wavelength that excites the donor is irradiated to the modified polypeptide when the donor is a fluorescent compound or a fluorescent protein, or a luminescent substrate for the donor is added to the modified polypeptide when the donor is a bioluminescent protein. Subsequently, detection of fluorescence from the modified polypeptide, specifically, detection of fluorescence from the modified peptide at the emission wavelength of the donor and/or detection of fluorescence from the modified peptide at the emission wavelength of the acceptor generated by Förster resonance energy transfer is performed.

When the lung cancer cell is sensitive to the TKI, in the absence of the TKI, fluorescence from the acceptor is detected by irradiating light of a wavelength that excites the donor or by adding a luminescent substrate for the donor, since the modified polypeptide is phosphorylated by EGFR and Förster resonance energy transfer occurs. On the other hand, in the presence of the TKI, fluorescence from the donor is detected by irradiating light of a wavelength that excites the donor or by adding a luminescent substrate for the donor, since phosphorylation of the modified polypeptide by EGFR is inhibited and thus Förster resonance energy transfer is suppressed.

In contrast, when the lung cancer cell is resistant to the TKI, since the modified polypeptide is phosphorylated by EGFR and Förster resonance energy transfer occurs regardless of the presence or absence of the TKI, fluorescence from the acceptor is detected.

Therefore, in step c), when intensity of fluorescence at the emission wavelength of the acceptor detected in step b) is lower than intensity of fluorescence from the modified polypeptide at said wavelength in the absence of the TKI, the lung cancer cell can be evaluated as being sensitive to the TKI, and when the intensity of fluorescence at the emission wavelength of the acceptor detected in step b) is equal to or higher than the intensity of fluorescence from the modified polypeptide at said wavelength in the absence of the TKI, the lung cancer cell can be evaluated as being resistant to the TKI.

Further, in step c), when intensity of fluorescence at the emission wavelength of the donor detected in step b) is higher than intensity of fluorescence from the modified polypeptide at said wavelength in the absence of the TKI, the lung cancer cell can be evaluated as being sensitive to the TKI, and when the intensity of fluorescence at the emission wavelength of the donor detected in step b) is equal to or lower than the intensity of fluorescence from the modified polypeptide at said wavelength in the absence of the TKI, the lung cancer cell can be evaluated as being resistant to the TKI.

Furthermore, in step c), when a ratio of intensity of fluorescence at the emission wavelength of the acceptor to intensity of fluorescence at the emission wavelength of the donor detected in step b) is lower than a ratio of intensity of fluorescence at the emission wavelength of the acceptor to intensity of fluorescence at the emission wavelength of the donor in the absence of the TKI, the lung cancer cell can be evaluated as being sensitive to the TKI, and when the ratio of intensity of fluorescence at the emission wavelength of the acceptor to intensity of fluorescence at the emission wavelength of the donor detected in step b) is equal to or higher than the ratio of intensity of fluorescence at the emission wavelength of the acceptor to intensity of fluorescence at the emission wavelength of the donor in the absence of the TKI, the lung cancer cell can be evaluated as being resistant to the TKI.

For example, when a ratio of intensity of fluorescence at the emission wavelength of the acceptor to intensity of fluorescence at the emission wavelength of the donor detected in step b) is lower by 3% or more, preferably lower by 5% or more, more preferably lower by 7% or more, still more preferably lower by 10% or more than a ratio of intensity of fluorescence at the emission wavelength of the acceptor to intensity of fluorescence at the emission wavelength of the donor in the absence of the TKI, the lung cancer cell can be evaluated as being sensitive to the TKI. For example, when a ratio of intensity of fluorescence at the emission wavelength of the acceptor to intensity of fluorescence at the emission wavelength of the donor detected in step b) is equal to or higher than, or is lower by less than 3% than, a ratio of intensity of fluorescence at the emission wavelength of the acceptor to intensity of fluorescence at the emission wavelength of the donor in the absence of the TKI, the lung cancer cell can be evaluated as being resistant to the TKI.

In step c) of the above-described embodiments, evaluation can be performed by comparison between fluorescence intensity or fluorescence intensity ratio in the presence of the TKI and a predetermined cutoff value instead of comparison between fluorescence intensity or fluorescence intensity ratio in the presence of the TKI and fluorescence intensity or fluorescence intensity ratio in the absence of the TKI. The cutoff value can be arbitrarily set in a range having sensitivity and specificity at or above levels generally required in clinical testing, based on fluorescence intensity or fluorescence intensity ratio obtained by subjecting TKI-sensitive lung cancer cells and TKI-resistant lung cancer cells to steps a) and b). The cutoff value can be set, for example, in a range where sensitivity is 80% or more and specificity is 80% or more. In a preferred embodiment, the cutoff value can be set by methods commonly used for determining cutoff values from ROC curves, for example, a method of setting the cutoff value at a point where Youden's index (sensitivity-(1-specificity)) is maximum, or a method of setting the cutoff value at a point where distance from the upper left corner of the ROC curve is minimum.

When using a cutoff value set based on fluorescence intensity ratio, for example, the fluorescence intensity ratio in the presence of the TKI can be compared with the cutoff value, and when the fluorescence intensity ratio in the presence of the TKI is lower than the cutoff value, the lung cancer cell can be evaluated as being sensitive to the TKI, and when the fluorescence intensity ratio is equal to or higher than the cutoff value, the lung cancer cell can be evaluated as being resistant to the TKI.

According to the method for evaluating sensitivity to the TKI of the present disclosure, sensitivity to the TKI of individual cells in a cell population containing lung cancer cells obtained from a lung cancer patient, particularly in a cell population containing non-small cell lung cancer cells from a lung cancer patient, can be evaluated, thereby enabling detection of a small number of TKI-resistant cells contained in said cell population. Furthermore, according to the method for evaluating sensitivity to the TKI of the present disclosure, a possibility of the TKI being effective in a lung cancer patient can be evaluated using, as an index, a proportion of TKI-resistant cells in a cell population containing lung cancer cells obtained from the lung cancer patient.

Evaluation of a possibility of the TKI being effective can be performed, for example, as follows. First, a cell population containing lung cancer cells obtained from a lung cancer patient is subjected to steps a) and b) both in the presence and absence of the TKI. Then, in step c), sensitivity to the TKI of individual cells in the cell population is evaluated by comparison with a predetermined cutoff value, and a proportion of cells evaluated as being resistant to the TKI (TKI-resistant cells) to the total number of cells in the cell population is calculated. When the proportion of TKI-resistant cells detected in the presence of the TKI is lower than the proportion of TKI-resistant cells detected in the absence of the TKI, the lung cancer patient can be evaluated as being sensitive to the TKI and having a high possibility of the TKI being effective. Further, when the proportion of TKI-resistant cells detected in the presence of the TKI is equal to or higher than the proportion of TKI-resistant cells detected in the absence of the TKI, the lung cancer patient can be evaluated as being resistant to the TKI and having a low possibility of the TKI being effective.

Thus, the method for evaluating sensitivity to the TKI of the present disclosure can also be used for determining applicability of the TKI to a lung cancer patient. Further, the reagent for measuring EGFR tyrosine kinase activity of the present disclosure can also be used as a diagnostic agent for determining applicability of the TKI to a lung cancer patient.

### Method for Preparing TKI-Resistant Lung Cancer Cell

In one aspect, the present disclosure provides a method for preparing a TKI-resistant lung cancer cell, particularly a TKI-resistant non-small cell lung cancer cell, including the steps of: a) contacting the above-described modified polypeptide with EGFR of the lung cancer cell in the presence of the TKI; b) detecting fluorescence from the modified polypeptide; c) evaluating sensitivity to the TKI based on the fluorescence; and d) collecting a cell evaluated as being resistant to the TKI. Steps a) to c) are as described above. In one embodiment, steps a) to d) are performed in vitro or ex vivo.

Step d) can be performed by collecting a cell evaluated as being resistant to the TKI in step c) with known cell separation means such as a fluorescence activated cell sorter (FACS). The TKI-resistant lung cancer cell prepared by this method can be suitably utilized in analysis of TKI-resistance mutations and research and development of new therapeutic agents for lung cancer.

### Method for Treating Lung Cancer

In one aspect, the present disclosure provides a method for treating lung cancer in a subject, including the above-described steps a) to c), and step e) administering the TKI to a lung cancer patient having a lung cancer cell evaluated as being sensitive to the TKI, or to a lung cancer patient evaluated as having a high possibility of the TKI being effective.

The modified polypeptide of the present disclosure can be produced and used with reference to the description in this specification as well as Japanese Patents No. 5665262 and No. 6473080, Mizutani, T. et al., Clinical Cancer Research 16, 3964-3975 (2010), and Horiguchi, M. et al., Cell Structure and Function 42 (1), 15-26 (2017). The descriptions in these documents are incorporated herein by reference in their entirety. The following examples specifically describe the present invention, but these examples are intended to facilitate understanding of the present invention and do not limit the technical scope of the present invention.

### [Examples]

### Materials and Methods

### (1) Reagents

Epidermal growth factor (EGF) was purchased from Peprotech, EGFR-TKI AG1478 from Calbiochem, Gefitinib from Wako Pure Chemical Industries, and Erlotinib and Afatinib from Selleck Chemicals. Anti-EGFR antibody was purchased from Atlas Antibodies AB, anti-c-Met (C-28) antibody and anti-actin antibody from Santa Cruz Technology, and anti-phospho-EGFR (Tyr1173) antibody (53A5), anti-phospho-Met antibody (Tyr1234/1235), anti-phospho-Akt antibody (S473), and anti-Akt antibody from Cell Signaling Technology. The TKIs used are shown in Table 1. The IC₅₀ values in the table represent 50% inhibitory concentrations against wild-type targets unless otherwise specified.

**[Table 1]**

| **Name (Trade name or others)** | **Target** | **IC₅₀** |
|---|---|---|
| AG-1478 (Tyrphostin AG-1478) | EGFR | 3 nM |
| Afatinib (Tomtovok, Tovok, BIBW2992) | EGFR | EGFR: WT, 0.5 nM; L858R, 0.4 nM; L858R/T790M, 10 nM |
| | HER2 | HER2: 14 nM |
| Gefitinib (Iressa, ZD-1839) | EGFR | 37 nM |
| Erlotinib Hydrochloride (Tarceva, CP-358774, OSI-774, NSC 718781) | EGFR | 2 nM |

### (2) Cell Lines and Cell Culture

The non-small cell lung cancer cell lines used are shown in Table 2. HCC827, PC3, and PC9 harbor EGFR-TKI-sensitive mutations, while H1299 has an EGFR-TKI-resistant mutation. In addition, Cos-1 (CRL-1650), a kidney epithelial cell line derived from African green monkey, was used.

**[Table 2]**

| **Cell line** | **Origin** | **Histology** | **Race** | **Sex** | **EGFR mutation** | **Other mutations** |
|---|---|---|---|---|---|---|
| A549 | Lung | Adenocarcinoma | Caucasian | Male | Wild-type | K-Ras (G12S) |
| H1299 | Lymph node | Large cell carcinoma | Caucasian | Male | Wild-type | p53 partial deletion |
| H1975 | Lung | Adenocarcinoma | Caucasian | Female | L858R, T790M | |
| HCC827 | Lung | Adenocarcinoma | Caucasian | Female | E746-A750del | |
| PC-9 | Lung | Adenocarcinoma | Japanese | | E746-A750del | |
| PC-3 | | Adenocarcinoma | | | L747-E749del, A750P | |

Non-small cell lung cancer cells were cultured in Roswell Park Memorial Institute (RPMI) 1640 medium supplemented with 10% fetal bovine serum (FBS) and 1% penicillin-streptomycin at 37°C in a humidified atmosphere containing 5% CO₂. Cos-1 cells were cultured in Dulbecco's Modified Eagle Medium (DMEM, Sigma) supplemented with 10% fetal bovine serum and 1% penicillin-streptomycin.

### (3) Expression Vectors

Expression vectors containing DNA encoding the following polypeptides were prepared according to Horiguchi, M. et al., Cell Structure and Function 42 (1), 15-26 (2017):
- Pickles_2.3 (SEQ ID NO: 4): A polypeptide containing, from the N-terminus, the amino acid sequence of Venus as an acceptor, the amino acid sequence corresponding to residues 1-222 of CrkL, and the amino acid sequence of cp173-ECFP as a donor.
- Pickles_2.31 (SEQ ID NO: 5): A polypeptide containing, from the N-terminus, the amino acid sequence of m1Venus as an acceptor, the amino acid sequence corresponding to residues 1-222 of CrkL, and the amino acid sequence of cp173-ECFP as a donor.
- Pickles_2.34 (SEQ ID NO: 6): A polypeptide containing, from the N-terminus, the amino acid sequence of m1Venus as an acceptor, the amino acid sequence corresponding to residues 1-222 of CrkL with aspartic acid at position 94 substituted with alanine, and the amino acid sequence of cp173-ECFP as a donor.
- Pickles_2.34NES (SEQ ID NO: 7): A polypeptide containing, from the N-terminus, the amino acid sequence of NES from protein kinase A inhibitor, the amino acid sequence of m1Venus as an acceptor, the amino acid sequence corresponding to residues 1-222 of CrkL with aspartic acid at position 94 substituted with alanine, and the amino acid sequence of cp173-ECFP as a donor. This polypeptide is Pickles_2.34 with the NES from protein kinase A inhibitor added to its N-terminus.

### (4) Gene Introduction

Transfection of expression vectors into cells was performed using Lipofectamine^{®} 2000 Transfection Reagent (Life Technology) or Lipofectamine^{®} 3000 Transfection Reagent (Life Technology) according to the manufacturer's protocol. In Example 3, polyethylenimine (PEI) (Polysciences), FuGene HD^{®} (Promega), and electroporation were also used according to the manufacturers' protocols. For PEI-mediated transfection, 6 µg of plasmid and 2 µl of PEI were mixed in 250 µl of Opti-MEM (Life Technology), vortexed, incubated for 5 minutes, and then added to the cells. For electroporation, nucleofection (Lonza) program X-001 and Solution V were used.

### (5) Fluorescence Microscopy

Fluorescence images of cells were acquired using an IX81 research-grade inverted microscope (Olympus) equipped with a stage incubator (LCI), a motorized XY-stage (Chuo Precision Industrial), and a CoolSNAP Myo cooled charge-coupled device (CCD) camera (Nippon Roper) under the control of MetaMorph software (Molecular Devices Japan). An XF1071 (440AF21) excitation filter, XF2034 (455DRLP) dichroic mirror, and fluorescence filters XF3075 (480AF30, for CFP) and XF3079 (535AF26, for FRET) corresponding to donor and acceptor fluorescence wavelengths at 480 nm and 535 nm, respectively (all from Omega Optical Inc.) were used. White light from a SOLA light engine (Lumencore) was attenuated by a 20% neutral density filter and used as excitation light after interference by each filter. Images were captured using a 60× oil immersion objective lens (numerical aperture 1.40) (4×4 binning, fluorescence images: exposure time 200 msec, differential interference contrast images: exposure time 20 msec).

### (6) Statistical Analysis

Data were subjected to one-way analysis of variance followed by Student's t-test. P < 0.01 was considered significant and is indicated by an asterisk in the figures.

### Example 1. Detection of EGFR Tyrosine Kinase Activity

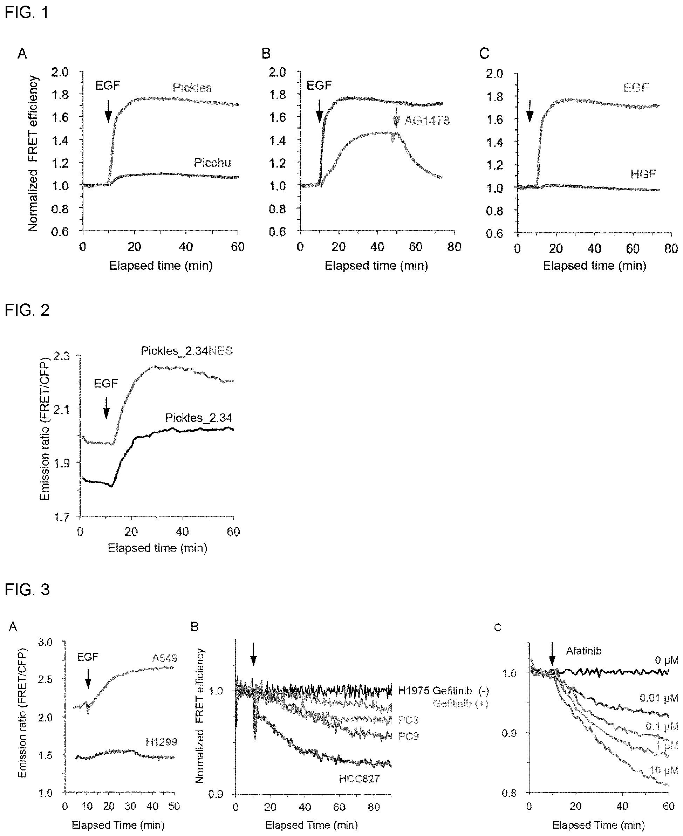

(1) pFX-Pickles_2.34, an expression vector for Pickles_2.34, or an expression vector for the Crkll biosensor Picchu (Kurokawa, K. et al., Journal of Biological Chemistry 276, 31305-31310 (2001)) was introduced into Cos-1 cells plated in 35-mm glass-bottom dishes. After 24 hours, the medium was changed to phenol red-free MEM-F12 medium, and the cells were serum-starved for 4 hours. Time-lapse fluorescence imaging was then initiated, with FRET, CFP, and DIC images acquired every 30 seconds. Cells were stimulated by adding EGF to a final concentration of 50 ng/ml at 10 minutes after starting imaging, and imaging was continued for another 50 minutes. Following background subtraction, FRET efficiency was calculated as the ratio of cellular fluorescence intensity (FRET/CFP) from each channel at each time point.
   In cells expressing Pickles, FRET efficiency significantly increased upon EGF stimulation. The increase in FRET efficiency upon EGF stimulation in cells expressing Pickles (77.7%) was markedly higher than that upon EGF stimulation in cells expressing Picchu (14.2%) (Fig. 1A).
(2) Using Cos-1 cells expressing Pickles, FRET efficiency was evaluated following the same procedure as in (1), except that the EGFR-TKI AG1478 (1 µM) was further added 40 minutes after EGF stimulation. Addition of EGFR-TKI to cells expressing Pickles resulted in a decrease in FRET efficiency (Fig. 1B).
(3) Using Cos-1 cells expressing Pickles, FRET efficiency was evaluated following the same procedure as in (1), except that EGF stimulation was replaced with the same amount of HGF stimulation. FRET efficiency in cells expressing Pickles did not change with HGF stimulation (Fig. 1C), demonstrating that Pickles can selectively detect EGFR signaling.

### Example 2. Detection of EGFR Tyrosine Kinase Activity in Lung Cancer Cells

(1) pFX-Pickles_2.34, an expression vector for Pickles_2.34, or pFX-Pickles_2.34NES, an expression vector for Pickles_2.34NES, was introduced into HCC827 cells, and FRET efficiency was evaluated following the same procedure as in Example 1(1). Pickles_2.34NES was confirmed to respond to EGF stimulation with higher sensitivity and show increased FRET efficiency compared to Pickles_2.34, which lacks NES (Fig. 2).
(2) pFX-Pickles_2.34NES was introduced into A549 cells and H1299 cells, and FRET efficiency was evaluated following the same procedure as in Example 1(1). While FRET efficiency increased significantly in A549 cells, the increase in FRET efficiency was slight in H1299 cells (Fig. 3A). It has been reported that EGFR expression is relatively high in A549 cells, while it is below the detection limit of Western blotting in H1299 cells (Li, Z. et al., Molecular Carcinogenesis, 51, 522-534 (2012)). Therefore, the difference in FRET efficiency before and after EGF stimulation is thought to reflect the difference in EGFR expression levels.
(3) pFX-Pickles_2.34NES was introduced into HCC827 cells, PC3 cells, PC9 cells, and H1975 cells, and FRET efficiency was evaluated following the same procedure as in Example 1(1), except that Gefitinib (37 nM), an EGFR-TKI, was added instead of EGF. Various degrees of decrease in FRET efficiency were observed depending on the cell line, whereas FRET efficiency showed little decrease in H1299 cells harboring EGFR-TKI-resistant mutation (Fig. 3B).
(4) pFX-Pickles_2.34NES was introduced into HCC827 cells, and FRET efficiency was evaluated following the same procedure as in Example 1(1), except that Afatinib (0, 0.01 µM, 0.1 µM, 1 µM, 10 µM) was added instead of EGF. FRET efficiency decreased in an Afatinib concentration-dependent manner (Fig. 3C), demonstrating that Pickles_2.34NES can quantitatively evaluate the effects of EGFR-TKIs.

These results confirmed that Pickles2.34NES can be used for detecting sensitivity for EGFR-TKI, detecting lung cancer cells harboring TKI-resistant mutations, and quantitatively determining effects of TKI inhibitor.

### Example 3. Comparison of Transfection Methods

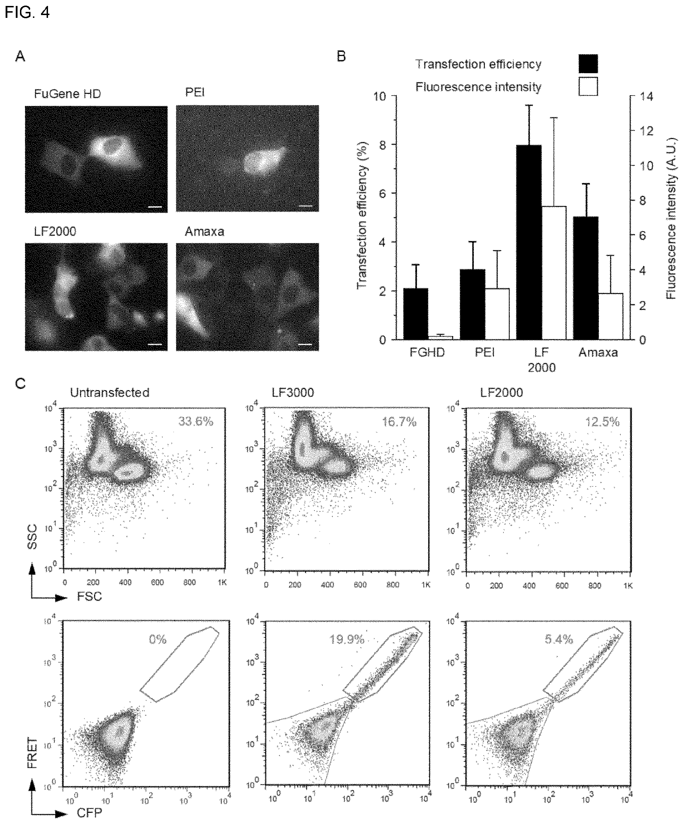

pFX-Pickles_2.34NES was introduced into HCC827 cells using FuGene HD, polyethylenimine, Lipofectamine^{®} 2000, or electroporation, and the cells were observed under a fluorescence microscope after 24 hours. Transfection using Lipofectamine^{®} 2000 showed the highest efficiency (approximately 8%) and high fluorescence intensity per cell (Fig. 4A and B).

pFX-Pickles_2.34NES was introduced into PC3 cells using either Lipofectamine^{®} 2000 or Lipofectamine^{®} 3000, and after 24 hours, the cells were detached and analyzed by flow cytometry together with untransfected cells. Transfection using Lipofectamine^{®} 3000 showed a 4-fold higher transfection efficiency compared to Lipofectamine^{®} 2000, and the cell survival rate was approximately 1.3-fold higher (Fig. 4C).

These results confirmed that for transfection of expression vectors into the cells, the use of either Lipofectamine^{®} 2000 or Lipofectamine^{®} 3000, particularly Lipofectamine^{®} 3000, is preferred. In subsequent experiments, Lipofectamine^{®} 3000 was used for transfection of expression vectors into the cells.

### Example 4. Isolation of EGFR-TKI-Resistant Cells Using FACS

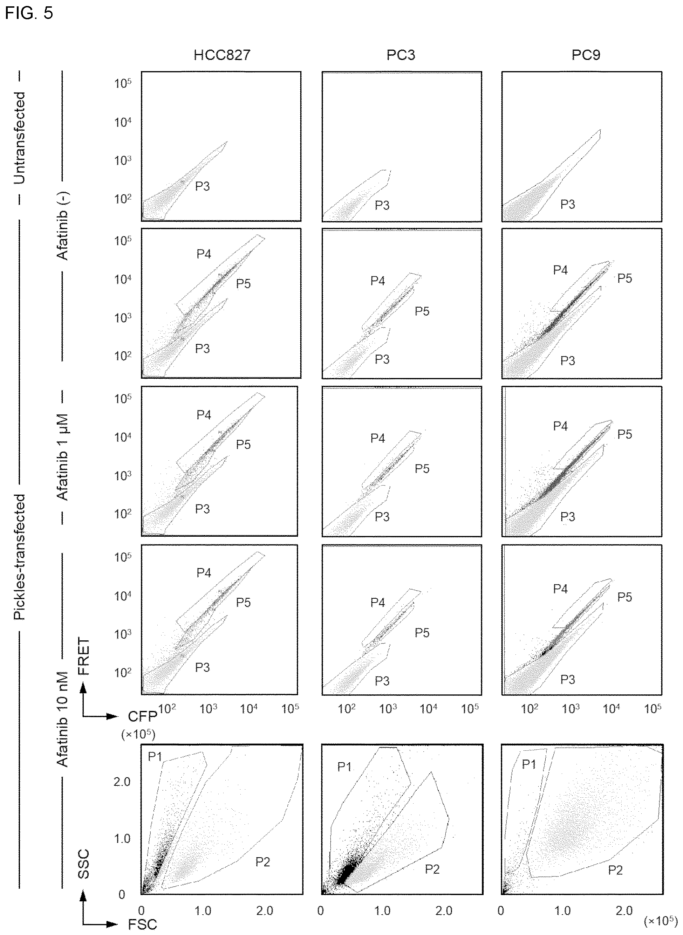

pFX-Pickles_2.34NES was introduced into PC3 cells, PC9 cells, and HCC827 cells, and after 24 hours, the cells were treated with 0, 10 nM, and 1 µM Afatinib for 2 hours. The treated cells were subjected to FACS analysis using BD FACSAriall (BD Biosciences). Cells were excited using a 405LD laser, and CFP and FRET signals were detected by the PacificBlue-A and AlexaFluor430-A channels, respectively. First, cells without Pickles introduction and without Afatinib treatment were analyzed to determine the region where Pickles non-expressing cells are distributed (Fig. 5, top panel, P3). Next, cells with Pickles introduction but without Afatinib treatment were analyzed to confirm the region where Pickles-expressing cells are distributed (Fig. 5, second panel). Then, FRET^{low}, the region where cells treated with 1 µM Afatinib are distributed, and FRET^{high}, the region where Afatinib-treated cells are not distributed, were defined (Fig. 5, third panel). Subsequently, cells introduced with Pickles and treated with 10 nM Afatinib were analyzed to distinguish viable cells (P1) from dead cells (P2) in FSC-SSC plots (Fig. 5, fifth panel), and cells in FRET^{high} (P4) and FRET^{low} (P5) after drug treatment were collected as H cells and L cells, respectively. H cells were expected to be Afatinib-resistant, while L cells were expected to be Afatinib-sensitive. The yields were 3.0×10⁵ H cells and 2.6×10⁴ L cells from PC3 cells, 6.2×10⁴ H cells and 7.9×10⁴ L cells from PC9 cells, and 1.8×10⁵ H cells and 6×10⁴ L cells from HCC827 cells, with sorting accuracy above 99%.

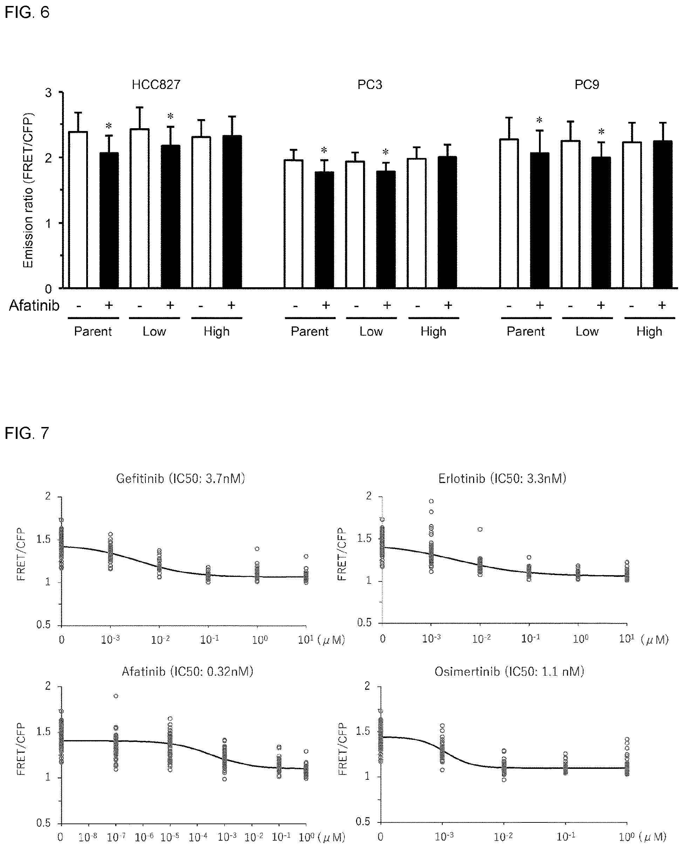

The isolated H cells and L cells, along with their respective parent strains, were treated with 10 nM Afatinib for 2 hours. FRET efficiency was calculated from FRET and CFP images of approximately 100 cells each before and after treatment. For all PC3 cells, PC9 cells, and HCC827 cells, the parent strains and L cells showed a decrease in FRET efficiency upon Afatinib treatment, whereas H cells maintained constant FRET efficiency after treatment (Fig. 6), confirming that H cells were Afatinib-resistant.

These results demonstrated that Pickles2.34NES can be used to detect and isolate TKI-resistant cells from a TKI-sensitive cell population.

### Example 5. Measurement of EGFR-TKI IC₅₀ Values

pFX-Pickles_2.34NES was introduced into HCC827 cells, and FRET efficiency was evaluated following the same procedure as in Example 1(1), except that EGFR-TKIs were added instead of EGF: Gefitinib (0, 10⁻³ µM, 10⁻² µM, 10⁻¹ µM, 1 µM, 10 µM), Erlotinib (0, 10⁻³ µM, 10⁻² µM, 10⁻¹ µM, 1 µM, 10 µM), Afatinib (0, 10⁻⁷ µM, 10⁻⁵ µM, 10⁻³ µM, 10⁻¹ µM, 1 µM), or Osimertinib (0, 10⁻³ µM, 10⁻² µM, 10⁻¹ µM, 1 µM). Dose-response curves of FRET efficiency versus EGFR-TKI treatment concentration were created using four-parameter logistic regression, and IC₅₀ values for each EGFR-TKI were determined (Fig. 7)."

The IC₅₀ values for EGFR-TKIs were comparable to previously reported values for EGFR with E746_A750del mutation (Gefitinib 4.8 nM, Erlotinib 4.9 nM, Afatinib 0.9 nM, Osimertinib 1.1 nM) (Kobayashi Y. et al., Cancer Science. 2016 Sep;107(9):1179-86. doi: 10.1111/cas.12996. Table 2), demonstrating that Pickles2.34NES can be used to evaluate EGFR-TKI activity with high sensitivity and in a short time period.

## Claims

1. A reagent for measuring a tyrosine kinase activity of epidermal growth factor receptor in a non-small cell lung cancer cell, comprising:
a modified polypeptide comprising a donor and an acceptor that induce Förster resonance energy transfer bound to CrkL, a CrkL fragment having an SH2 domain and a portion subjected to phosphorylation by a tyrosine kinase, or a CrkL variant consisting of an amino acid sequence having an identity of 90% or more with the amino acid sequence of CrkL or the CrkL fragment and acting as a substrate for a tyrosine kinase; or
a nucleic acid encoding the modified polypeptide.

2. The reagent according to claim 1, wherein the CrkL is a polypeptide consisting of the amino acid sequence represented by SEQ **ID** NO: 1.

3. The reagent according to claim 1, wherein the CrkL fragment is a polypeptide consisting of an amino acid sequence in which 1 to 81 arbitrary amino acids are deleted from the C-terminus of the amino acid sequence represented by SEQ **ID** NO: 1.

4. The reagent according to claim 1, wherein the CrkL variant is any one of the following polypeptides (1) to (3):
(1) a polypeptide consisting of an amino acid sequence in which at least one amino acid of amino acids at positions 91 to 97 in the amino acid sequence represented by SEQ **ID** NO: 1 is substituted;
(2) a polypeptide consisting of an amino acid sequence in which 1 to 81 arbitrary amino acids are deleted from the C-terminus of the amino acid sequence represented by SEQ **ID** NO: 1, and at least one amino acid of amino acids at positions 91 to 97 is substituted; or
(3) a polypeptide consisting of an amino acid sequence having an identity of 90% or more with the amino acid sequence of the polypeptide of (1) or (2), and having suppressed cleavage compared to a polypeptide consisting of an amino acid sequence in which amino acids at positions 91 to 97 are not substituted, wherein the polypeptide acts as a substrate for a tyrosine kinase.

5. The reagent according to claim 4, wherein the substitution of at least one amino acid of amino acids at positions 91 to 97 is a substitution of the amino acid at position 94.

6. The reagent according to claim 4, wherein the substitution of at least one amino acid of amino acids at positions 91 to 97 is a substitution of the amino acid at position 94 with alanine.

7. The reagent according to claim 1, wherein the donor and the acceptor are fluorescent proteins.

8. The reagent according to claim 7, wherein the modified polypeptide has a structure in which a fluorescent protein as the donor is bound, directly or via a linker sequence, to one terminus of the CrkL, CrkL fragment, or CrkL variant, and a fluorescent protein as the acceptor is bound, directly or via a linker sequence, to the other terminus.

9. The reagent according to claim 7, wherein the donor is CFP or a variant thereof, and the acceptor is YFP or a variant thereof.

10. The reagent according to claim 7, wherein the donor is ECFP, and the acceptor is m1Venus.

11. The reagent according to claim 1, wherein the modified polypeptide further comprises a nuclear export signal.

12. The reagent according to claim 1, wherein the modified polypeptide has an amino acid sequence represented by any one of SEQ ID NOs: 4 to 7.

13. A method for evaluating sensitivity of a non-small cell lung cancer cell to a tyrosine kinase inhibitor, comprising the steps of:
(a) contacting the modified polypeptide as defined in any one of claims 1 to 12 with EGFR of the non-small cell lung cancer cell in the presence of the tyrosine kinase inhibitor;
(b) detecting fluorescence from the modified polypeptide; and
(c) evaluating sensitivity to the tyrosine kinase inhibitor based on the fluorescence.

14. The method according to claim 13, wherein step (a) is performed within the non-small cell lung cancer cell.

15. The method according to claim 13, wherein step (a) is performed by incubating a non-small cell lung cancer cell introduced with a nucleic acid encoding the modified polypeptide in the presence of the tyrosine kinase inhibitor.

16. The method according to claim 15, wherein introduction of the nucleic acid is performed by lipofection.

17. The method according to claim 13, wherein in step (b), fluorescence from the modified polypeptide is detected at emission wavelengths of the donor and the acceptor, and in step (c), when a ratio of fluorescence intensity at the emission wavelength of the acceptor to fluorescence intensity at the emission wavelength of the donor is lower by 3% or more than a ratio of fluorescence intensity at the emission wavelength of the acceptor to fluorescence intensity at the emission wavelength of the donor in the absence of the tyrosine kinase inhibitor, the non-small cell lung cancer cell is evaluated as being sensitive to the tyrosine kinase inhibitor.

18. A method for preparing a tyrosine kinase inhibitor-resistant non-small cell lung cancer cell, comprising the steps of:
(a) contacting the modified polypeptide as defined in any one of claims 1 to 12 with **EGFR** of the non-small cell lung cancer cell in the presence of the tyrosine kinase inhibitor;
(b) detecting fluorescence from the modified polypeptide;
(c) evaluating sensitivity to the tyrosine kinase inhibitor based on the fluorescence; and
(d) collecting a cell evaluated as being resistant to the tyrosine kinase inhibitor.

19. A method for measuring a tyrosine kinase activity of **EGFR** in a non-small cell lung cancer cell, comprising the steps of:
(a') contacting the modified polypeptide as defined in any one of claims 1 to 12 with **EGFR** of the non-small cell lung cancer cell; and
(b) detecting fluorescence from the modified polypeptide.
